# EUROPEAN PATENT SPECIFICATION

(11) **EP 3 640 643 B1**
(45) Date of publication and mention of the grant of the patent: **22.12.2021**
(21) Application number: 19201633.5
(22) Date of filing: 24.01.2017
(51) Int. Cl.: G01N 33/574

(54) **METHOD FOR PREDICTING THE OUTCOME OF A TREATMENT WITH AFLIBERCEPT OF A PATIENT SUSPECTED TO SUFFER FROM A CANCER BY MEASURING THE LEVEL OF A PLASMA BIOMARKER**
VERFAHREN ZUR VORHERSAGE DES AUSGANGS EINER BEHANDLUNG MIT AFLIBERCEPT EINES PATIENTEN MIT VERDACHT AUF KREBSLEIDEN DURCH MESSUNG DES PLASMABIOMARKERSPIEGELS
PROCÉDÉ PERMETTANT DE PRÉDIRE LE RÉSULTAT D'UN TRAITEMENT AVEC AFLIBERCEPT D'UN PATIENT SUSPECTÉ DE SOUFFRIR D'UN CANCER PAR MESURE DU NIVEAU D'UN BIOMARQUEUR DE PLASMA

(30) Priority: 25.01.2016 EP 16305065
(43) Date of publication of application: 22.04.2020
(62) Divisional of application: 17701686.2
(73) Proprietor: Sanofi, 75008 Paris (FR)
(72) Inventor: DREYMANN, Jennifer, 75008 Paris (FR); PACCARD, Caroline, 75008 Paris (FR); CHIRON-BLONDEL, Marielle, 75008 Paris (FR)
(74) Representative: Sanofi

(56) References cited:
- WO-A1-2016/008975
- US-B1- 6 749 853
- DIETHER LAMBRECHTS ET AL: "Evaluation of efficacy and safety markers in a phase II study of metastatic colorectal cancer treated with aflibercept in the first-line setting", BRITISH JOURNAL OF CANCER, vol. 113, no. 7, 10 September 2015 (2015-09-10), pages 1027-1034, XP055283970, GB ISSN: 0007-0920, DOI: 10.1038/bjc.2015.329
- J. BAAR ET AL: "A Vasculature-Targeting Regimen of Preoperative Docetaxel with or without Bevacizumab for Locally Advanced Breast Cancer: Impact on Angiogenic Biomarkers", CLINICAL CANCER RESEARCH, vol. 15, no. 10, 5 May 2009 (2009-05-05), pages 3583-3590, XP055284319, US ISSN: 1078-0432, DOI: 10.1158/1078-0432.CCR-08-2917
- MARKUS WEHLAND ET AL: "Biomarkers for Anti-Angiogenic Therapy in Cancer", INTERNATIONAL JOURNAL OF MOLECULAR SCIENCES 2012 MDPI AG CHE, vol. 14, no. 5, 1 January 2013 (2013-01-01), pages 9338-9364, XP055283946, ISSN: 1661-6596, DOI: 10.3390/ijms14059338
- J. F. DE GROOT ET AL: "Myeloid Biomarkers Associated with Glioblastoma Response to Anti-VEGF Therapy with Aflibercept", CLINICAL CANCER RESEARCH, vol. 17, no. 14, 1 June 2011 (2011-06-01), pages 4872-4881, XP055367887, US ISSN: 1078-0432, DOI: 10.1158/1078-0432.CCR-11-0271
- M. CHIRON ET AL: "Differential Antitumor Activity of Aflibercept and Bevacizumab in Patient-Derived Xenograft Models of Colorectal Cancer", MOLECULAR CANCER THERAPEUTICS, vol. 13, no. 6, 31 March 2014 (2014-03-31) , pages 1636-1644, XP055367889, US ISSN: 1535-7163, DOI: 10.1158/1535-7163.MCT-13-0753
- CLAUS ZEHETNER ET AL: "Systemic Counterregulatory Response of Placental Growth Factor Levels to Intravitreal Aflibercept Therapy", INVESTIGATIVE OPTHALMOLOGY & VISUAL SCIENCE, vol. 56, no. 5, 21 May 2015 (2015-05-21), pages 3279-3286, XP055367860, US ISSN: 1552-5783, DOI: 10.1167/iovs.15-16686

## Description

The present invention concerns the use of PIGF as a biomarker for predicting the outcome of the treatment with aflibercept, or ziv-aflibercept, of a patient suspected to suffer from a colon cancer, a colorectal cancer or a rectal cancer.

Aflibercept, or ziv-aflibercept, also referred to as VEGFR1R2-Fc.DELTA.C1 Flt1D2.Flk1D3.Fc.DELTA.C1 or AVE0005, is a homo dimer protein, with each dimer comprising two identical monomers, each of which is a fusion protein comprising the signal sequence of VEGFR1 fused to the D2 Ig domain of the VEGFR1 receptor, itself fused to the D3 Ig domain of the VEGFR2 receptor, in turn fused to the Fc domain of IgG1.

The protein chain is glycosylated, with N-acetyl-glucosamine, fucose, galactose, mannose and sialic acids contributing to the carbohydrate structures. The N-linked oligosaccharides consist of mainly bi-antennary structures with zero, one or two terminal sialic acids. The monomer has the amino acid sequence SEQ ID N°1.

The U.S. Food and Drug Administration (FDA) already approved aflibercept under the trade name EYLEA^{®} for the treatment of patients with neovascular (wet) age-related macular degeneration (AMD). In particular, EYLEA^{®} is the trade name for aflibercept as generated, processed and formulated for intravitreal injection.

At the time of registration of aflibercept (ZALTRAP^{®}) for cancer indication, and In light of aflibercept's approved use in treating AMD, the FDA requested that a different name (ziv-aflibercept) be given for the compound's use in the treatment of cancer. Thus, ziv-aflibercept is the United States Adopted Name (USAN) accepted by FDA to designate a pharmaceutical composition comprising aflibercept as generated, processed and formulated for injection via intravenous infusion. Ziv-aflibercept has been approved by the FDA for sale under the tradename ZALTRAP^{®} for the treatment of metastatic colorectal cancer (mCRC).

The European Medicines Agency (EMA) approved ZALTRAP^{®} as well however did not request separate names for the compound. Thus, in the European Union the name "aflibercept" is used regardless of the indication.

ZALTRAP^{®} and EYLEA^{®} are obtained by slightly different processes. They both contain aflibercept or ziv-aflibercept, but the ratio of aggregates of aflibercept or ziv-aflibercept is slightly different in ZALTRAP^{®} and EYLEA^{®}.

ZALTRAP^{®} approval was based on data obtained from the VELOUR trial - a multicenter, randomized, placebo-controlled phase III trial, which compared the efficacy of aflibercept *versus* placebo in combination with the FOLFIRI regimen for patients with mCRC previously treated with an oxaliplatin containing regimen.

Despite the efficacy and the safety of the treatment of cancer by aflibercept it remains a goal to better identify patients who should benefit more from the treatment. Indeed the ability to identify Metastatic Colorectal Cancer (mCRC) patients who will benefit from aflibercept would further improve clinical utility of this drug.

To date, no validated predictive serum or plasma biomarkers have been identified that correlate with treatment outcomes to aflibercept.

The profiling of tumor and plasma samples derived from patients involved in clinical trials and subsequent analysis of their genomic / proteomic and clinical data could allow the discovery and potential validation of predictive biomarkers.

It has now been discovered that high levels of VCAM-1, ICAM-1 and/or PIGF at baseline correlated with shorter survival times.

Vascular cell adhesion molecule-1 (VCAM-1) also known as CD106 has the sequence SEQ ID N°1 (NCBI Reference Sequence: NP_001069.1). The term "VCAM-1" encompasses its homologues and isoforms and variants thereof, as well as fragments of the sequences, provided that the variant proteins (including isoforms), homologous proteins and/or fragments are recognized by one or more VCAM-1 specific antibodies.

Intercellular Adhesion Molecule 1 (ICAM-1) also known as CD54 has the sequence SEQ ID N°2 (NCBI Reference Sequence: NP_000192.2). The term "ICAM-1" encompasses its homologues and isoforms and variants thereof, as well as fragments of the sequences, provided that the variant proteins (including isoforms), homologous proteins and/or fragments are recognized by one or more ICAM-1 specific antibodies.

"Placental Growth Factor" or "PIGF" comprises the 2 isoforms PIGF1 et PIGF2 which have respectively the sequences SEQ ID N°3 (NCBI Reference Sequence: NP_002623.2) and SEQ ID N°4 (NCBI Reference Sequence: NP_001193941.1). The term " PIGF " encompasses PIGF1 et PIGF2 their homologues and isoforms and variants thereof, as well as fragments of the sequences, provided that the variant proteins (including isoforms), homologous proteins and/or fragments are recognized by one or more PIGF specific antibodies.

The correlation of ICAM-1 with patient outcome was tested in clinical trials where patients were treated with bevacizumab.

The correlation of ICAM-1 with patient outcome was found in a trial wherein lung cancer patients were treated with bevacizumab, cisplatin and etoposide (Horn et al, J Clin Oncol 2009; 27:6006-6011). The authors found that "patients who had high ICAM levels had a non significant trend towards improved OS compared with patients who had low levels", whereas in another study (Dowlati et al, 2008, Clin Cancer Res 14(5), 1407) "Patients with low baseline ICAM had a higher response than those with high ICAM".

But the correlation of ICAM-1 with patient outcome was not found in colorectal cancer patients treated with bevacizumab, capecitabine and oxaliplatin (Liu et al; Cancer Medicine 2013; 2(2): 234-242).

These articles show the level of unpredictability between various biomarkers studies with the same biologics i.e. bevacizumab.

Furthermore bevacizumab is an anti-VEGF-A antibody. Aflibercept is not an antibody but a chimeric protein. It consists of portions of extracellular domains of human VEGF receptors 1 and 2 fused to human IgG1 Fc portion. Aflibercept binds not only to VEGF-A but also to VEGF-B and placental growth factor (PIGF).

Thus bevacizumab and aflibercept have structure and functions that are very different and the man skilled in the art would not transpose directly the results obtained with bevacizumab to aflibercept.

The correlation of VCAM-1 with patient outcome was found in the two clinical trials mentioned above (Liu et al; Cancer Medicine 2013; 2(2): 234-242 and Horn et al, J Clin Oncol 2009; 27:6006-6011). However in these two studies there is not a placebo arm to let us know if the effect is predictive or prognostic.

Wehland et al. (Int J Mol Sci. 2013 May; 14(5): 9338-9364) is a review on biomarkers for anti-angiogenic therapy in Cancer, concluding that no general or cancer-specific biomarker has yet emerged, which could help select patients with a positive prognosis for anti-angiogenic therapy.

De Groot et al. (Clin Cancer Res. 2011 July 15; 17(14): 4872―4881) reports compelling evidence that elevated levels of myeloid-associated chemokines such as MIF, IP-10, MCP3/CCL7, and CTACK/ CCL27 are predictive of radiographic response, and that a decrease in VEGFR1+ monocytes from baseline to 24 hours was associated with response, both in in patients with glioblastoma.

Chiron et al. (Mol Cancer Ther. 2014 Jun;13(6):1636-44) indicates that aflibercept was more active in a greater number of colorectal cancer Patient-Derived Xenograft (PDX) models than bevacizumab.

Zehetner et al. (Invest Ophthalmol Vis Sci. 2015 May;56(5):3279-86) reports a significant systemic upregulation of the proangiogenic cytokine PIGF after intravitreal administration of aflibercept, and that might represent a counter-regulatory response to antiangiogenic therapy.

Lambrechts et al. (British Journal of Cancer (2015) 113, 1027-1034) describes that high IL8 plasma levels at baseline is associated with worse PFS in patients having metastatic colorectal cancer and treated with aflibercept, suggesting that IL8 may act as a potentially predictive biomarker of aflibercept treatment outcome. WO2016008975 discloses the use of interleukin-8 (IL-8) as a biomarker for predicting the outcome of the treatment with aflibercept, or ziv-aflibercept of a patient suspected to suffer from a cancer.

The invention relates to the use of PIGF as a biomarker for predicting the outcome of the treatment with aflibercept, or ziv-aflibercept of a patient suspected to suffer from a colon cancer, a colorectal cancer or a rectal cancer.

In one aspect, the present invention provides a method of determining whether a patient suspected to suffer from a colon cancer, a colorectal cancer or a rectal cancer is a candidate for aflibercept, or ziv-aflibercept therapy for the said cancer comprising the step of subjecting a patient's biological sample to at least one assay to measure at baseline the level of PIGF, wherein when the biological sample of the biomarker level is low relative to a reference level of expression of PIGF, the patient is identified as a candidate for therapy for cancer.

In another aspect, the present invention provides a method of determining whether a patient suspected to suffer from a colon cancer, a colorectal cancer or a rectal cancer is a candidate for aflibercept, or ziv-aflibercept therapy for the said cancer comprising the step of subjecting a patient's biological sample to at least one assay to measure at baseline the level of PIGF, wherein when the biological sample level of the biomarker is high relative to a reference level of expression of PIGF, the patient is identified as not being a candidate for therapy for cancer. The threshold or reference level of expression of PIGF allows to define sensitive and non-sensitive populations.

In an embodiment the reference level of expression of PIGF is comprised between around 12 ng/mL and around 19 ng/mL. Yet in another embodiment the reference level of expression of PIGF is around 17 ng/mL.

The invention relates also to a method for treating a patient with a colon cancer, a colorectal cancer or a rectal cancer with aflibercept, or ziv-aflibercept, comprising administering a therapeutically effective amount of aflibercept, or ziv-aflibercept to the patient, wherein the level of PIGF in the patient's biological sample is low relative to a reference level of expression of PIGF.

Yet in another aspect the invention relates to a method for improving the progression- free survival (PFS) and/or the overall survival (OS) of a patient with a colon cancer, a colorectal cancer or a rectal cancer, comprising administering a therapeutically effective amount of aflibercept, or ziv-aflibercept to the patient, wherein the level of PIGF in the patient's biological sample is low relative to a reference level of expression of PIGF.

In an embodiment of one of the methods described above the biological sample is chosen from the group consisting of blood, serum and plasma.

In an embodiment the colorectal cancer is a metastatic colorectal cancer.

In another embodiment of the invention, the subject is treated with aflibercept and further undergoes a chemotherapeutic treatment with oxaliplatin, 5-fluorouracil (5-FU) and folinic acid (i.e. the FOLFOX treatment), folinic acid, 5-fluorouracil and irinocetan (i.e. the FOLFIRI treatment), or 5-fluorouracil and folinic acid (i.e. the FUFOL or LV5FU2 treatment).

The chemotherapeutic treatment may combine at least 2, 3, 4, 5, 6, 7, 8, 9, 10 or at most 10, 9, 8, 7, 6, 5, 4, 3, 2 agents, such as e.g. a combination of oxaliplatin, 5-fluorouracil (5-FU) and folinic acid (i.e. the FOLFOX treatment or the modified FOLFOX6 treatment as described in the example below), a combination of folinic acid, 5-fluorouracil and irinocetan (i.e. the FOLFIRI treatment), or a combination of 5-fluorouracil and folinic acid (i.e. the FUFOL or LV5FU2 treatment).

In this regard the application WO2012146610 relates to a method of treatment of the mCRC by aflibercept, or ziv-aflibercept in combination with FOLFIRI.

In an embodiment of one of the methods described above therapeutically effective amounts of aflibercept, or ziv-aflibercept, oxaliplatin, 5-fluorouracil (5-FU) and folinic acid are administered to said patient.

In an embodiment of one of the methods described above therapeutically effective amounts of aflibercept, or ziv-aflibercept, folinic acid, 5- fluorouracil (5-FU) and irinocetan are administered to said patient.

In a further embodiment of one of the methods described above folinic acid at a dosage comprised between about 200 mg/m² and about 600 mg/m², 5-fluorouracil (5-FU) at a dosage comprised between about 2000 mg/m² and about 4000 mg/m², irinocetan at a dosage comprised between about 100 mg/m² and about 300 mg/m² and aflibercept at a dosage comprised between about 1 mg/kg and about 10 mg/kg are administered to patient.

In a further embodiment of one of the methods described above folinic acid at a dosage of about 400 mg/m², 5-fluorouracil (5-FU) at a dosage of about 2800 mg/m², irinocetan at a dosage of about 180 mg/m² and aflibercept at a dosage of about 4 mg/kg are administered to patient.

In a further embodiment of one of the methods described above folinic acid is administered intravenously at a dosage of about 400 mg/m², 5-fluorouracil (5-FU) is administered intravenously at a dosage of about 2800 mg/m², irinocetan is administered intravenously at a dosage of about 180 mg/m² and aflibercept is administered intravenously at a dosage of about 4 mg/kg and wherein the combination is administered every two weeks.

In a further embodiment of one of the methods described above folinic acid, 5-fluorouracil (5-FU), irinocetan and aflibercept are administered intravenously every two weeks for a period comprised between 9 and 18 weeks.

In a further embodiment of one of the methods described above folinic acid is administered intravenously immediately after aflibercept administration. It can be also administered intravenously immediately after aflibercept administration over a period of about 2 hours.

In a further embodiment of one of the methods described above irinocetan is administered intravenously immediately after aflibercept administration. It can be also administered intravenously immediately after aflibercept administration over a period of about 90 minutes.

In a further embodiment of one of the methods described above 5-fluorouracil (5-FU) is administered immediately after aflibercept administration.

In a further embodiment of one of the methods described above a first quantity of 5-fluorouracil (5-FU) is administered intravenously immediately after aflibercept administration and a second quantity of 5-FU is administered intravenously after the first quantity in continuous infusion.

In a further embodiment of one of the methods described above about 400 mg/m² of 5- fluorouracil (5-FU) is administered intravenously over a period of 2 to 4 minutes after aflibercept administration and wherein 2400 mg/m² of 5-FU is administered intravenously over around 46 hours after the administration of the 400 mg/m² in continuous infusion

In an embodiment said patient has previously been treated with therapy based on oxaliplatin or on bevacizumab.

In another embodiment said patient has failed with chemotherapy, radiotherapy or surgery.

The invention relates also to aflibercept, or ziv-aflibercept for use in treating a patient suspected to suffer from cancer, wherein the patient has been identified as having lower level of a PIGF in biological sample as compared to the reference level of expression of PIGF.

The above methods and use of the invention may be, for instance, in vitro or ex vivo methods and use.

Means for measuring the expression level of VCAM-1, ICAM-1 and PIGF protein are well-known in the art and include immunoassay such as ELISA assay. For instance the means for measuring VCAM-1 include antibodies specifically binding to VCAM-1.

The level of VCAM-1 protein may be, for instance, determined using immunological detection methods such as an ELISA assay. The methods involve an antibody which binds to VCAM-1 protein, for example a monoclonal or polyclonal antibody, an antibody variant or fragments such as a single chain antibody, a diabody, a minibody, a single chain Fv fragment (sc(Fv)), a Sc(Fv)2 antibody, a Fab fragment or a F(ab')2 fragment, or a single domain antibody. Such antibodies are well known in the art and are commercially available. They may also notably be obtained by immunization of animals (for example rabbits, rats or mice) with VCAM-1 protein. Antibodies may be used to determine protein expression in a range of immunological assays including competitive and non-competitive assay systems using techniques such as western blotting, immunohistochemistry/ immunofluorescence (i.e protein detection on fixed cells or tissues), radioimmunoassay such as RIA (radio-linked immunoassay), ELISA (enzyme linked immunosorbent assay), "sandwich" immunoassays, immunoprecipitation assays, immunodiffusion assays, agglutination assays, complement-fixation assays, immunoradiometric assays, fluorescent immunoassays, e.g. FIA (fluorescence-linked immunoassay), chemiluminescence immunoassays, ECLIA (electrochemiluminescence immunoassay) and protein A immunoassays. Such assays are routine and well known to the person skilled in the art (Ausubel et al (1994) Current Protocols in Molecular Biology, Vol. 1, John Wiley & Sons, Inc., New York).

Protein expression of VCAM-1 may be determined by proteomic method such as mass spectrometry assays (LC-MS or LC-MS/MS). Qualitative and quantitative mass spectrometric techniques are known and used in the art. To this aim, target peptides specific for marker proteins are selected and quantified based on calibration curves established with synthetic peptides labeled with stable isotopes. Enzymatic digests, spiked with a defined amount of isotope labeled target peptides, are analyzed by liquid chromatography coupled with mass spectrometry. The ratio between labeled and non-labeled target peptides is measured to assess target peptide concentrations and therefore protein marker concentration.

The means for measuring the expression level of VCAM-1 may also include reagents such as e.g. reaction and/or washing buffers. The means may be present, e.g., in vials or microtiter plates, or be attached to a solid support such as a microarray as can be the case for primers and probes.

Similar means are at the disposal of the man skilled in the art for detecting ICAM-1 and PIGF.

In an embodiment VCAM-1, ICAM-1 and PIGF proteins can be measured with a bead-based multiplex assay, the Luminex^{™} technology.

Aflibercept, or ziv-aflibercept is provided in a formulation which is not prejudicial to the patient to be treated.

In an embodiment aflibercept, or ziv-aflibercept is provided in a formulation with sucrose and polysorbate 20 (stabilisers), sodium chloride, citrate buffer, and sodium phosphate buffer, adjusted to final pH.

In another embodiment aflibercept, or ziv-aflibercept, is supplied in two drug product presentations:
- a presentation at 100 mg aflibercept, or ziv-aflibercept / 4.0 mL (nominal concentration).
- a second presentation at 200 mg aflibercept, or ziv-aflibercept / 8.0 mL (nominal concentration).

Both presentations are manufactured from the same bulk sterile solution at 25 mg/mL of aflibercept, or ziv-aflibercept.

Prior to infusion to the patient, the concentrate solution is diluted with 0.9% sodium chloride solution or 5% dextrose.

The anti-cancer agents used in the above recited method or use are provided in a pharmaceutically acceptable carrier, excipient or diluent which is not prejudicial to the patient to be treated.

Pharmaceutically acceptable carriers and excipient that may be used in the compositions of this invention include, but are not limited to, ion exchangers, alumina, aluminium stearate, lecithin, self-emulsifying drug delivery systems (SEDDS) such as d-a-tocopherol polyethyleneglycol 1000 succinate, surfactants used in pharmaceutical dosage forms such as Tweens or other similar polymeric delivery matrices, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

As appreciated by skilled artisans, compositions are suitably formulated to be compatible with the intended route of administration. Examples of suitable routes of administration include parenteral route, including for instance intramuscular, subcutaneous, intravenous, intraperitoneal or local intratumoral injections. The oral route can also be used, provided that the composition is in a form suitable for oral administration, able to protect the active principle from the gastric and intestinal enzymes.

The term "effective amount" refers to an amount of a drug alone or in combination with other drug or treatment regimen effective to treat a disease or disorder in a mammal. In the case of cancer, the therapeutically effective amount of the drug may reduce the number of cancer cells; reduce the tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy in vivo can, for example, be measured by assessing the duration of survival, duration of progression free survival (PFS), the response rates (RR), duration of response, and/or quality of life.

The terms "Therapy", "therapeutic", "treatment" and "treating" are used herein to characterize a therapeutic method or process that is aimed at (1) slowing down or stopping the progression, aggravation, or deterioration of the symptoms of the disease state or condition to which such term applies; (2) alleviating or bringing about ameliorations of the symptoms of the disease state or condition to which such term applies; and/or (3) reversing or curing the disease state or condition to which such term applies.

The term "overall survival (OS)" refers to the length of time during and after treatment the patient survives. As the skilled person will appreciate, a patient's overall survival is improved or enhanced, if the patient belongs to a subgroup of patients that has a statistically significant longer mean survival time as compared to another subgroup of patients.

The term "progression- free survival (PFS)" refers to the length of time during and after treatment during which, according to the assessment of the treating physician or investigator, the patient's disease does not become worse, i.e., does not progress. As the skilled person will appreciate, a patient's progression- free survival is improved or enhanced if the patient belongs to a subgroup of patients that has a longer length of time during which the disease does not progress as compared to the average or mean progression free survival time of a control group of similarly situated patients.

A "subject" or a "patient" may be a human or a non-human mammal, such as monkeys, dogs, cats, guinea pigs, hamsters, rabbits, cows, horses, goats and sheep.

The term "reference level" herein refers to a predetermined value. As the skilled artisan will appreciate the reference level is predetermined and set to meet the requirements in terms of e.g. specificity and/or sensitivity. These requirements can vary, e.g. from regulatory body to regulatory body. It may for example be that assay sensitivity or specificity, respectively, has to be set to certain limits, e.g. 80%, 90% or 95%. These requirements may also be defined in terms of positive or negative predictive values. Nonetheless, based on the teaching given in the present invention it will always be possible to arrive at the reference level meeting those requirements. In one embodiment the reference level is determined in healthy individuals. The reference value in one embodiment has been predetermined in the disease entity to which the patient belongs. In certain embodiments the reference level can e.g. be set to any percentage between 25% and 75% of the overall distribution of the values in a disease entity investigated. In other embodiments the reference level can e.g. be set to the median, tertiles or quartiles as determined from the overall distribution of the values in a disease entity investigated. In one embodiment the reference level is set to the median value as determined from the overall distribution of the values in a disease entity investigated.

### BRIEF DESCRIPTION OF THE DRAWINGS

Figure 1 represents Kaplan-Meier curves for PFS endpoint for sensitive and non-sensitive populations defined with VCAM-1.
Figure 2 represents Kaplan-Meier curves for OS endpoint for sensitive and non-sensitive populations defined with VCAM-1.
Figure 3 represents Kaplan-Meier curves for PFS endpoint for sensitive and non-sensitive populations defined with ICAM-1.
Figure 4 represents Kaplan-Meier curves for OS endpoint for sensitive and non-sensitive populations defined with ICAM-1.
Figure 5 represents Kaplan-Meier curves for PFS endpoint for sensitive and non-sensitive populations defined with VCAM-1 and ICAM-1.
Figure 6 represents Kaplan-Meier curves for OS endpoint for sensitive and non-sensitive populations defined with VCAM-1 and ICAM-1.
Figure 7 represents Kaplan-Meier curves for PFS endpoint for sensitive and non-sensitive populations defined with PIGF.

### EXAMPLE: Predictive effect of PIGF on PFS in the AFLAME study

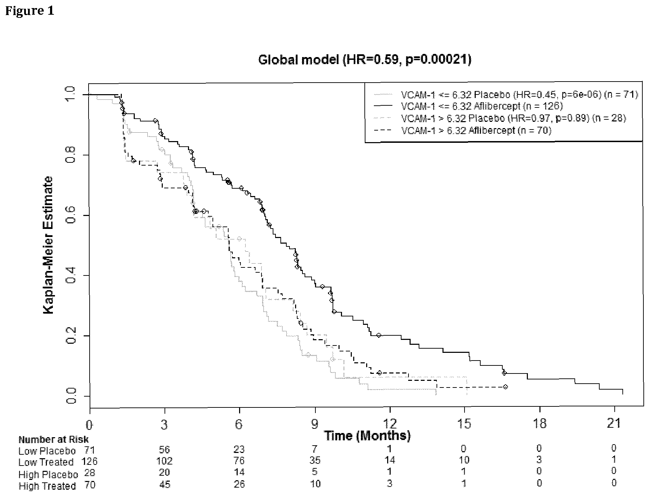

Aspects on VCAM-1 and ICAM-1 (including Figures 1 to 6) are embodiments of the study EFC11338 (AFLAME) described below, but are embodiments not covered by the claimed invention.

### STUDY EFC11338 (AFLAME)

EFC11338 was designed as a Multinational, Randomized, Double-Blind Study of Aflibercept Versus Placebo with Irinotecan/5-FU Combination (FOLFIRI) in Patients with Metastatic Colorectal Cancer (MCRC) After Failure of an Oxaliplatin Based Regimen.

### Objectives:

To identify potential predictive biomarkers for response to treatment on efficacy endpoints (Progression Free Survival (PFS), Overall Survival (OS) and Overall Response Rate (ORR))
To identify potential prognostic biomarkers for efficacy endpoints (PFS, OS and ORR)
To identify potential correlation between biomarkers and other baseline characteristics
To identify potential correlation of longitudinal plasma measurements with clinical endpoints
To identify potential groups of homogeneous individuals based on their molecular profiles in an unsupervised way and to estimate correlation with clinical outcomes (PFS, OS and ORR)
To assess the safety profile of the population identified by prognostic/predictive biomarkers

### Dosage and schedule of administration:

Patients were administered either aflibercept or placebo, depending on arm assigned. Immediately after, patients received irinotecan, 5-FU and leucovorin (FOLFIRI regimen). This treatment was repeated every 2 weeks.

### Aflibercept/placebo

Arm A, aflibercept: 4 mg/kg was administered IV over 1 hour on Day 1, every 2 weeks,
   OR
Arm B, placebo: 4 mg/kg was administered IV over 1 hour on Day 1, every 2 weeks.

### FOLFIRI regimen

Immediately after aflibercept/placebo administration, all the patients received:
- Irinotecan 180 mg/m2 IV infusion in 500 mL D5W over 90 minutes and dl leucovorin* 400 mg/m2 IV infusion over 2 hours, at the same time, in bags using a Y-line, followed by:
- 5-FU 400 mg/m2 IV bolus given over 2-4 minutes, followed by:
- 5-FU 2400 mg/m2 continuous IV infusion in 500 mL D5W (recommended)
over 46-hours.

### Duration of treatment:

Patient was treated until disease progression, unacceptable toxicity or patient's refusal

### Duration of observation:

Patients were followed when on study treatment and during follow up period until death or the study cut-off date for OS, whichever comes first. The cut-off date for OS is one year after the last patient enrolled.

### Number of subjects:

Intent-to-Treat (ITT) population: 332 (109 in the placebo group + 223 in the aflibercept group)
Evaluable population for response rate: 295 (96 in the placebo group + 199 in the aflibercept group)
Evaluable for Luminex biomarkers - Group 1 biomarkers: 295 (99 in the placebo group + 196 in the aflibercept group)
Evaluable for Luminex biomarkers - Group 2 biomarkers: 108 (37 in the placebo group + 71 in the aflibercept group)
Evaluable for ELISA biomarkers: 329 (107 in the placebo group + 222 in the aflibercept group)

Due to an operational error, some patients randomized to the aflibercept arm have received placebo for one or several treatment cycles; or vice versa. A total of 198 patients received at least one misallocated treatment kit.

### Criteria for evaluation:

Biomarkers:
Different types of biomarker data were investigated in the current translational research proposal in the AFLAME study:
- 107 plasma angiogenic factors and inflammatory cytokines measured at baseline, during and after treatment (end of cycle 1 infusion, 48h post aflibercept/placebo on cycle 2 or 3, 30 days after last aflibercept/placebo administration) with Luminex^{®} technology
- Free VEGF-A and PIGF measured in plasma samples at baseline with ELISA (Enzyme-Linked Immunosorbent Assay) technology

### PREPARATION OF SAMPLES AND ANALYSIS

Plasma biomarkers were measured either with Luminex^{™} technology (bead-based multiplex assay) or with ELISA.

### Plasma biomarkers measured with Luminex^{™} technology

Plasma angiogenic factors and inflammatory cytokines have been measured at baseline pre cycle 1.

Thirty proteins measured on all the samples were defined as Group 1 biomarkers and 77 proteins measured only for some samples were defined as Group 2 biomarkers. The biomarkers from Group 1 were angiogenic/inflammatory molecules and have been selected based on aflibercept mechanism of action (inhibiting 3 angiogenic factors and their receptors), key candidate biomarkers identified on independent aflibercept studies or literature/experts.

### Plasma biomarkers measured with ELISA

In addition to plasma biomarkers measured with Luminex^{™} technology, free VEGF-A and free PIGF concentrations of baseline plasma samples were measured with ELISA technology.

### RESULTS

### Univariate analysis

Biomarkers have been tested for predictive and prognostic effects for PFS.

VCAM-1 and ICAM-1 have been identified as potentially predictive with corrected Benjamini-Hochberg (BH) p-value ≤ 0.2 (295 subjects).

Then sensitive and non-sensitive populations have been defined using VCAM-1 and ICAM-1 (BH p-value ≤ 0.2).

PIGF has been identified as potentially predictive by ELISA (unadjusted p-value = 0.075).

### Identification of sensitive and non-sensitive populations with VCAM-1

The threshold of 6.32 corresponding to 553 ng/mL has been determined to define sensitive (197 individuals) and non-sensitive populations (98 individuals) respectively corresponding to individuals with low values for VCAM-1 and high values for VCAM-1.

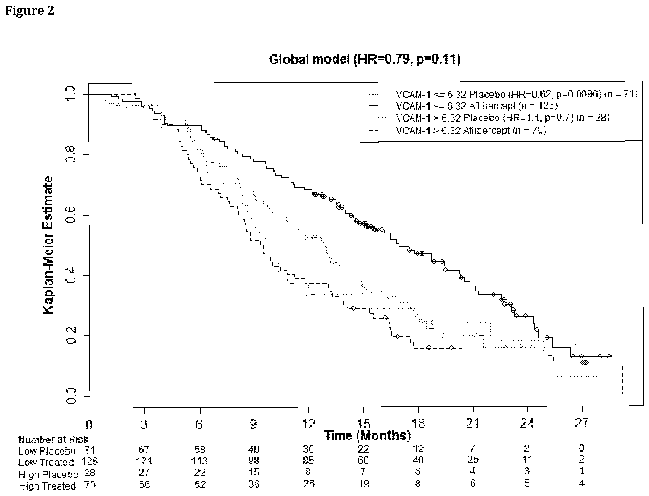

Figure 1 and Figure 2 represent Kaplan-Meier curves for PFS and OS endpoints for sensitive and non-sensitive populations illustrating the better treatment effect for low VCAM-1 group compared to high VCAM-1 group. For OS, in addition to a better treatment effect compared to placebo, sensitive population showed globally a better prognostic (increased OS for low VCAM-1 group in placebo and aflibercept arms).

Table 1 shows the response rate for sensitive and non-sensitive populations by treatment group. There was an increased response rate (26%) for the aflibercept/folfiri treatment in sensitive population compared to the non-sensitive population (10%).

**Table 1 - Response Rate for non-sensitive/sensitive populations defined with VCAM-1 for PFS - Preprocessed data - VCAM-1 and RR evaluable population**

| **Population** | **Placebo/Folfiri** | **Aflibercept/Folfiri** |
|---|---|---|
| Sensitive VCAM-1 <= 6.32 (N = 189) | 3/68 (4%) | 31/121 (26%) |
| Non-sensitive VCAM-1 > 6.32 (N = 94) | 1/26 (4%) | 7/68 (10%) |
| Total | 4/94 (4%) | 38/189 (20%) |

### Identification of sensitive and non-sensitive populations with ICAM-1

The threshold of 5.04 corresponding to 144 ng/mL has been determined to define sensitive (205 individuals) and non-sensitive populations (90 individuals) respectively corresponding to individuals with low values for ICAM-1 and high values for ICAM-1.

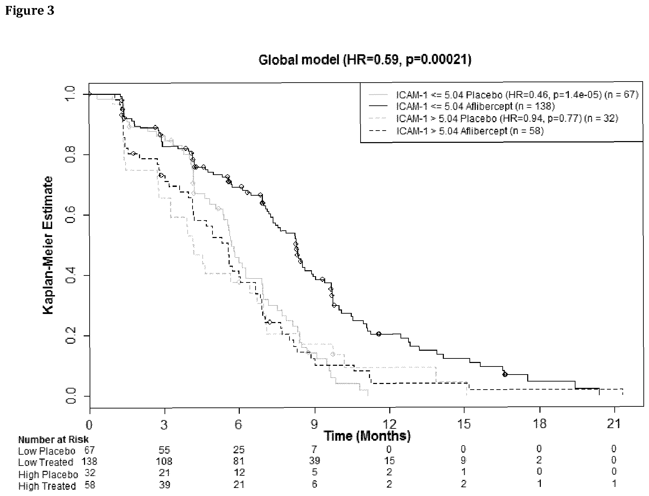

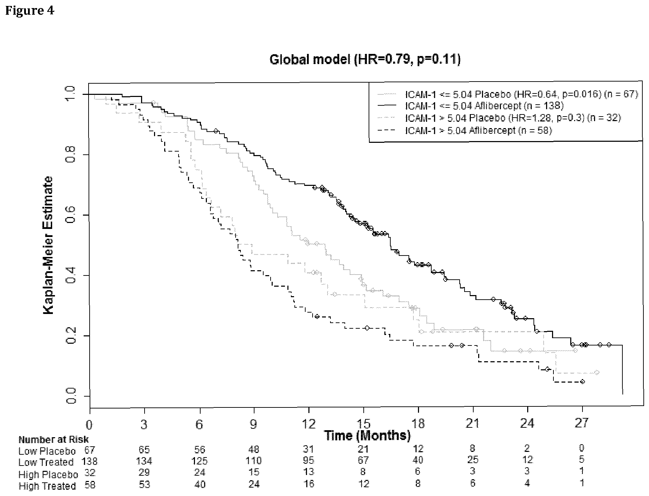

Figure 3 and Figure 4 represent Kaplan-Meier curves for PFS and OS endpoints for sensitive and non-sensitive populations illustrating the better treatment effect for low ICAM-1 group compared to high ICAM-1 group. For OS, in addition to a better treatment effect compared to placebo, sensitive population showed globally a better prognostic (increased OS for low ICAM-1 group in placebo and aflibercept arms).

Table 2 shows the response rate for sensitive and non-sensitive populations by treatment group. There has been an increased response rate (25%) for the aflibercept/folfiri treatment in sensitive population compared to the non-sensitive population (9%).

**Table 2 - Response Rate for non-sensitive/sensitive populations defined with ICAM-1 for PFS - Preprocessed data - ICAM-1 and RR evaluable population**

| **Population** | **Placebo/Folfiri** | **Aflibercept/Folfiri** |
|---|---|---|
| Sensitive ICAM-1 <= 5.04 (N = 197) | 3/63 (5%) | 33/134 (25%) |
| Non-sensitive ICAM-1 > 5.04 (N = 86) | 1/31 (3%) | 5/55 (9%) |
| Total | 4/94 (4%) | 38/189 (20%) |

### Multivariate analysis

The multivariate predictive score 0.089 x ICAM - 1 + 0.17 x VCAM - 1 has been dichotomized using quantile 10% to 90% as threshold.

Sensitive population showed a significant difference in PFS and in OS in favor of aflibercept over placebo (HR= 0.47 for PFS and HR = 0.66 for OS), which was increased compared to the non-sensitive population (HR= 0.98 for PFS and HR = 1.04 for OS).

Median PFS difference between aflibercept and placebo was equal to 2.59 months in sensitive population, showing a greater but moderate difference compared to non-sensitive population (0.5 month).

Median OS difference between aflibercept and placebo was equal to 3.75 months in sensitive population, showing a greater difference compared to non-sensitive population (-0.39 month). For non-sensitive population there was a decrease in median OS for placebo arm and treated arm (8.90 months and 8.51 months) compared to the other populations illustrating a potential prognostic effect of multivariate score in addition to the predictive effect.

In conclusion sensitive population showed a decreased HR compared to the non-sensitive population for PFS and OS with moderate gain in term of median.

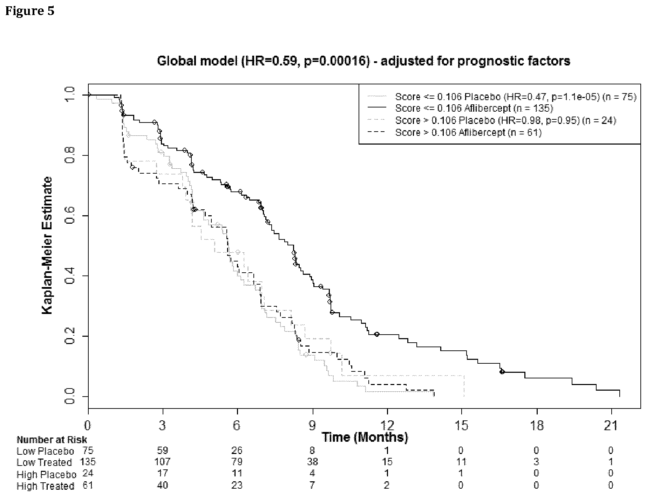

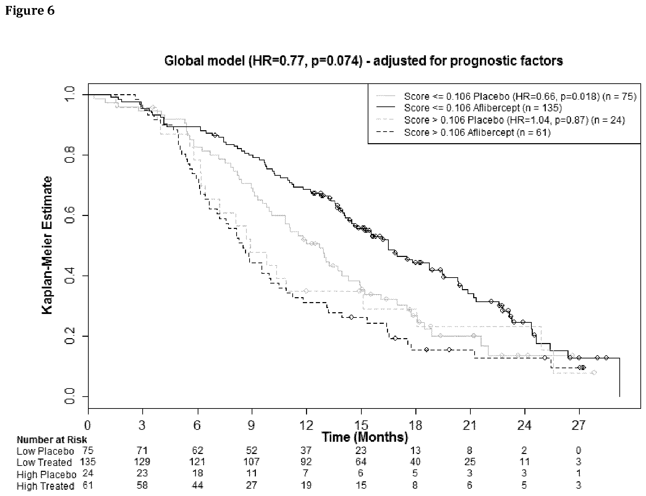

Figure 5 and Figure 6 represent Kaplan-Meier curves for PFS and OS endpoints for sensitive and non-sensitive populations illustrating the better treatment effect for low score group compared to high score group. For OS, in addition to a better treatment effect compared to placebo, sensitive population showed globally a better prognostic (increased OS for low score group in placebo and aflibercept arms).

### Identification of sensitive and non-sensitive populations with PIGF

The threshold of 2.82 corresponding to 17 pg/ml has been determined to define sensitive (230 individuals) and non-sensitive populations (99 individuals) respectively corresponding to individuals with low values for PIGF and high values for PIGF.

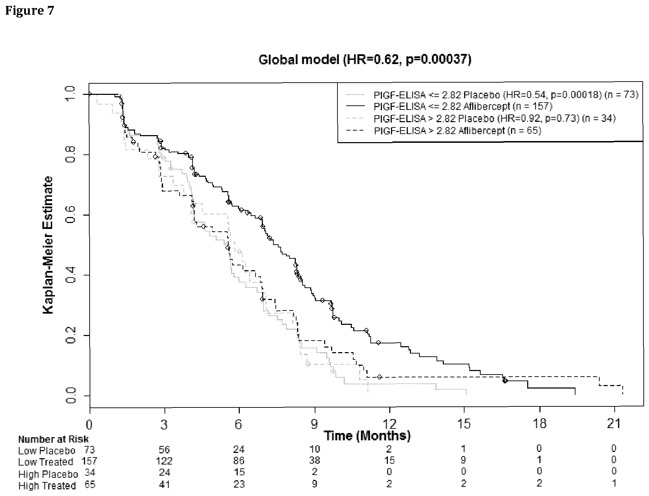

Figure 7 which represents the Kaplan-Meier curves for PFS endpoint for sensitive and non-sensitive populations defined with PIGF illustrates the determination of the PIGF cut off for sensitive and non-sensitive populations.

### CONCLUSIONS

- VCAM-1 and ICAM-1 have been identified as potentially predictive biomarkers for PFS in a univariate framework (unadjusted p-value equal to 0.00017 for VCAM-1 and 0.0043 for ICAM-1).
- The third biomarker that showed up to be potentially predictive was PIGF measured by ELISA (unadjusted p-value = 0.075).
- Linear combination of VCAM-1 and ICAM-1 has been identified as potentially predictive for PFS.

## Claims

1. Use of Placenta Growth Factor (PIGF) for predicting the outcome of the treatment with aflibercept of a patient suspected to suffer from a colon cancer, a colorectal cancer or a rectal cancer.

2. A method of determining whether a patient suspected to suffer from a colon cancer, a colorectal cancer or a rectal cancer is a candidate for aflibercept therapy for the said cancer comprising the step of subjecting a patient's biological sample to at least one assay to measure at baseline the level of PIGF, wherein when the PIGF level in the biological sample is low relative to a reference level of expression of PIGF, the patient is identified as a candidate for therapy for cancer.

3. A method of determining whether a patient suspected to suffer from a colon cancer, a colorectal cancer or a rectal cancer is a candidate for aflibercept therapy for the said cancer comprising the step of subjecting a patient's biological sample to at least one assay to measure at baseline the level of PIGF, wherein when the PIGF level in the biological sample is high relative to a reference level of expression of PIGF, the patient is identified as not being a candidate for therapy for cancer.

4. A method according to any of claims 2 and 3 wherein the reference level of expression of PIGF is between 12 and 19 pg/ml.

5. A method according to any of claims 2 to 4, wherein the reference level of expression of PIGF is 17 pg/ml.

6. A method according to any of claims 2 to 5, wherein the biological sample is chosen from the group consisting of blood, serum and plasma.

7. A method according to claim 6 wherein the colorectal cancer is a metastatic colorectal cancer.

8. A method according to any of claims 3 to 7, wherein the PIGF level which is determined is the circulating level.

9. Aflibercept for use in treating a patient suspected to suffer from a colon cancer, a colorectal cancer or a rectal cancer, wherein the patient has been identified as having lower level of PIGF in biological sample as compared to the reference level of expression of PIGF.

10. Combination comprising aflibercept, folinic acid, 5-fluorouracil (5-FU) and irinotecan for use in treating a patient suspected to suffer from a colon cancer, a colorectal cancer or a rectal cancer, wherein the patient has been identified as having a low level of PIGF in a biological sample compared to the reference level of expression of PIGF.

11. Combination comprising aflibercept, folinic acid, 5-fluorouracil (5-FU) and irinotecan for use according to claim 10 wherein the subject is treated with aflibercept and further undergoes a chemotherapeutic treatment with folinic acid, 5-fluorouracil and irinocetan.

12. Aflibercept or combination comprising aflibercept, folinic acid, 5-fluorouracil (5-FU) and irinotecan for use according to any of claims 9 to 11 wherein the reference level of expression of PIGF is between 12 and 19 pg/ml.

13. Aflibercept or combination comprising aflibercept, folinic acid, 5-fluorouracil (5-FU) and irinotecan for use according to claim 12 wherein the reference level of expression of PIGF is 17 pg/ml

14. Aflibercept or combination comprising aflibercept, folinic acid, 5-fluorouracil (5-FU) and irinotecan for use according to any one of claims 9 to 13 wherein the colorectal cancer is a metastatic colorectal cancer.

15. Combination comprising aflibercept, folinic acid, 5-fluorouracil (5-FU) and irinotecan for use according to any one of claims 9 to 14, wherein folinic acid at a dosage comprised between 200 mg/m² and 600 mg/m², 5-fluorouracil (5-FU) at a dosage comprised between 2000 mg/m² and 4000 mg/m², irinocetan at a dosage comprised between 100 mg/m² and 300 mg/m² and aflibercept at a dosage comprised between 1 mg/kg and 10 mg/kg are administered to patient.

## Patentansprüche

1. Verwendung von PlGF (Placenta Growth Factor) zur Vorhersage des Ergebnisses der Behandlung eines Patienten, bei dem ein Leiden an einem Kolonkarzinom, einem Kolorektalkarzinom oder einem Rektumkarzinom vermutet wird, mit Aflibercept.

2. Verfahren zur Bestimmung, ob ein Patient, bei dem ein Leiden an einem Kolonkarzinom, einem Kolorektalkarzinom oder einem Rektumkarzinom vermutet wird, ein Kandidat für eine Aflibercept-Therapie gegen die Krebserkrankung ist, umfassend den Schritt, bei dem eine biologische Probe des Patienten wenigstens einem Testverfahren zur Messung des Grundniveaus von PlGF unterzogen wird, wobei der Patient bei einem niedrigen PIGF-Niveau in der biologischen Probe relativ zu einem Referenzexpressionsniveau von PlGF als Kandidat für eine Krebstherapie identifiziert wird.

3. Verfahren zur Bestimmung, ob ein Patient, bei dem ein Leiden an einem Kolonkarzinom, einem Kolorektalkarzinom oder einem Rektumkarzinom vermutet wird, ein Kandidat für eine Aflibercept-Therapie gegen die Krebserkrankung ist, umfassend den Schritt, bei dem eine biologische Probe des Patienten wenigstens einem Testverfahren zur Messung des Grundniveaus von PlGF unterzogen wird, wobei der Patient bei einem hohen PIGF-Niveau in der biologischen Probe relativ zu einem Referenzexpressionsniveau von PlGF als kein Kandidat für eine Krebstherapie identifiziert wird.

4. Verfahren nach einem der Ansprüche 2 und 3, wobei das Referenzexpressionsniveau von PlGF zwischen 12 und 19 pg/ml liegt.

5. Verfahren nach einem der Ansprüche 2 bis 4, wobei das Referenzexpressionsniveau von PlGF bei 17 pg/ml liegt.

6. Verfahren nach einem der Ansprüche 2 bis 5, wobei die biologische Probe aus der Gruppe bestehend aus Blut, Serum und Plasma gewählt ist.

7. Verfahren nach Anspruch 6, wobei es sich bei dem Kolorektalkarzinom um ein metastatisches Kolorektalkarzinom handelt.

8. Verfahren nach einem der Ansprüche 3 bis 7, wobei es sich bei dem PIGF-Niveau, das bestimmt wird, um den Blutkreislaufspiegel handelt.

9. Aflibercept zur Verwendung bei der Behandlung eines Patienten, bei dem ein Leiden an einem Kolonkarzinom, einem Kolorektalkarzinom oder einem Rektumkarzinom vermutet wird, wobei bei dem Patienten das Vorliegen eines niedrigeren Niveaus von PlGF in einer biologischen Probe im Vergleich zu dem Referenzexpressionsniveau von PlGF identifiziert wurde.

10. Kombination, umfassend Aflibercept, Folinsäure, 5-Fluoruracil (5-FU) und Irinotecan, zur Verwendung bei der Behandlung eines Patienten, bei dem ein Leiden an einem Kolonkarzinom, einem Kolorektalkarzinom oder einem Rektumkarzinom vermutet wird, wobei bei dem Patienten das Vorliegen eines niedrigen Niveaus von PlGF in einer biologischen Probe verglichen mit dem Referenzexpressionsniveau von PlGF identifiziert wurde.

11. Kombination, umfassend Aflibercept, Folinsäure, 5-Fluoruracil (5-FU) und Irinotecan, zur Verwendung nach Anspruch 10, wobei das Individuum mit Aflibercept behandelt wird und sich ferner einer chemotherapeutischen Behandlung mit Folinsäure, 5-Fluoruracil und Irinocetan unterzieht.

12. Aflibercept oder Kombination, die Aflibercept, Folinsäure, 5-Fluoruracil (5-FU) und Irinotecan umfasst, zur Verwendung nach einem der Ansprüche 9 bis 11, wobei das Referenzexpressionsniveau von PlGF zwischen 12 und 19 pg/ml liegt.

13. Aflibercept oder Kombination, die Aflibercept, Folinsäure, 5-Fluoruracil (5-FU) und Irinotecan umfasst, zur Verwendung nach Anspruch 12, wobei das Referenzexpressionsniveau von PlGF bei 17 pg/ml liegt.

14. Aflibercept oder Kombination, die Aflibercept, Folinsäure, 5-Fluoruracil (5-FU) und Irinotecan umfasst, zur Verwendung nach einem der Ansprüche 9 bis 13, wobei es sich bei dem Kolorektalkarzinom um ein metastatisches Kolorektalkarzinom handelt.

15. Kombination, die Aflibercept, Folinsäure, 5-Fluoruracil (5-FU) und Irinotecan umfasst, zur Verwendung nach einem der Ansprüche 9 bis 14, wobei Folinsäure in einer Dosis, die zwischen 200 mg/m² und 600 mg/m² liegt, 5-Fluoruracil (5-FU) in einer Dosis, die zwischen 2000 mg/m² und 4000 mg/m² liegt, Irinocetan in einer Dosis, die zwischen 100 mg/m² und 300 mg/m² liegt, und Aflibercept in einer Dosis, die zwischen 1 mg/kg und 10 mg/kg liegt, einem Patienten verabreicht werden.

## Revendications

1. Utilisation du facteur de croissance placentaire (PlGF) pour la prédiction de l'issue du traitement avec de l'aflibercept chez un patient suspecté de souffrir d'un cancer du côlon, d'un cancer colorectal ou d'un cancer rectal.

2. Procédé pour déterminer si un patient suspecté de souffrir d'un cancer du côlon, d'un cancer colorectal ou d'un cancer rectal est un candidat pour une thérapie à l'aflibercept pour ledit cancer comprenant l'étape de soumission d'un échantillon biologique du patient à au moins une analyse pour mesurer avant le traitement le taux de PlGF, dans lequel, lorsque le taux de PlGF dans l'échantillon biologique est bas par rapport à un taux de référence d'expression de PlGF, le patient est identifié comme un candidat pour une thérapie pour le cancer.

3. Procédé pour déterminer si un patient suspecté de souffrir d'un cancer du côlon, d'un cancer colorectal ou d'un cancer rectal est un candidat pour une thérapie à l'aflibercept pour ledit cancer comprenant l'étape de soumission d'un échantillon biologique du patient à au moins une analyse pour mesurer avant le traitement le taux de PlGF, dans lequel, lorsque le taux de PlGF dans l'échantillon biologique est élevé par rapport à un taux de référence d'expression de PlGF, le patient est identifié comme n'étant pas un candidat pour une thérapie pour le cancer.

4. Procédé selon l'une quelconque des revendications 2 et 3, le taux de référence d'expression de PlGF étant compris entre 12 et 19 pg/ml.

5. Procédé selon l'une quelconque des revendications 2 à 4, le taux de référence d'expression de PlGF étant de 17 pg/ml.

6. Procédé selon l'une quelconque des revendications 2 à 5, l'échantillon biologique étant choisi dans le groupe constitué par le sang, le sérum et le plasma.

7. Procédé selon la revendication 6, le cancer colorectal étant un cancer colorectal métastatique.

8. Procédé selon l'une quelconque des revendications 3 à 7, le taux de PlGF qui est déterminé étant le taux circulant.

9. Aflibercept pour une utilisation dans le traitement d'un patient suspecté de souffrir d'un cancer du côlon, d'un cancer colorectal ou d'un cancer rectal, le patient ayant été identifié comme ayant un taux plus bas de PlGF dans l'échantillon biologique par rapport au taux de référence d'expression de PlGF.

10. Combinaison comprenant de l'aflibercept, de l'acide folinique, du 5-fluorouracile (5-FU) et de l'irinotécan pour une utilisation dans le traitement d'un patient suspecté de souffrir d'un cancer du côlon, d'un cancer colorectal ou d'un cancer rectal, le patient ayant été identifié comme ayant un taux bas de PlGF dans un échantillon biologique par rapport au taux de référence d'expression de PlGF.

11. Combinaison comprenant de l'aflibercept, de l'acide folinique, du 5-fluorouracile (5-FU) et de l'irinotécan pour une utilisation selon la revendication 10, le sujet étant traité avec de l'aflibercept et subissant en outre un traitement chimiothérapeutique avec de l'acide folinique, du 5-fluorouracile et de l'irinotécan.

12. Aflibercept ou combinaison comprenant de l'aflibercept, de l'acide folinique, du 5-fluorouracile (5-FU) et de l'irinotécan pour une utilisation selon l'une quelconque des revendications 9 à 11, le taux de référence d'expression de PlGF étant compris entre 12 et 19 pg/ml.

13. Aflibercept ou combinaison comprenant de l'aflibercept, de l'acide folinique, du 5-fluorouracile (5-FU) et de l'irinotécan pour une utilisation selon la revendication 12, le taux de référence d'expression de PlGF étant de 17 pg/ml.

14. Aflibercept ou combinaison comprenant de l'aflibercept, de l'acide folinique, du 5-fluorouracile (5-FU) et de l'irinotécan pour une utilisation selon l'une quelconque des revendications 9 à 13, le cancer colorectal étant un cancer colorectal métastatique.

15. Combinaison comprenant de l'aflibercept, de l'acide folinique, du 5-fluorouracile (5-FU) et de l'irinotécan pour une utilisation selon l'une quelconque des revendications 9 à 14, l'acide folinique, le 5-fluorouracile (5-FU), l'irinotécan et l'aflibercept étant administrés au patient respectivement à un dosage compris entre 200 mg/m² et 600 mg/m², un dosage compris entre 2 000 mg/m² et 4 000 mg/m², un dosage compris entre 100 mg/m² et 300 mg/m², et un dosage compris entre 1 mg/kg et 10 mg/kg.


### SEQUENCE LISTING

<110> SANOFI
<120> METHOD FOR PREDICTING THE OUTCOME OF A TREATMENT WITH AFLIBERCEPT OF A PATIENT SUSPECTED TO SUFFER FROM A CANCER BY MEASURING THE LEVEL OF A PLASMA BIOMARKER
<130> FR2015-020
<160> 4
<170> PatentIn version 3.5
<210> 1
   <211> 739
   <212> PRT
   <213> Homo sapiens
<400> 1
   Met Pro Gly Lys Met Val Val Ile Leu Gly Ala Ser Asn Ile Leu Trp
   1               5                   10                  15
   Ile Met Phe Ala Ala Ser Gln Ala Phe Lys Ile Glu Thr Thr Pro Glu
               20                  25                  30
   Ser Arg Tyr Leu Ala Gln Ile Gly Asp Ser Val Ser Leu Thr Cys Ser
           35                  40                  45
   Thr Thr Gly Cys Glu Ser Pro Phe Phe Ser Trp Arg Thr Gln Ile Asp
       50                  55                  60
   Ser Pro Leu Asn Gly Lys Val Thr Asn Glu Gly Thr Thr Ser Thr Leu
   65                  70                  75                  80
   Thr Met Asn Pro Val Ser Phe Gly Asn Glu His Ser Tyr Leu Cys Thr
                   85                  90                  95
   Ala Thr Cys Glu Ser Arg Lys Leu Glu Lys Gly Ile Gln Val Glu Ile
               100                 105                 110
   Tyr Ser Phe Pro Lys Asp Pro Glu Ile His Leu Ser Gly Pro Leu Glu
           115                 120                 125
   Ala Gly Lys Pro Ile Thr Val Lys Cys Ser Val Ala Asp Val Tyr Pro
       130                 135                 140
   Phe Asp Arg Leu Glu Ile Asp Leu Leu Lys Gly Asp His Leu Met Lys
   145                 150                 155                 160
   Ser Gln Glu Phe Leu Glu Asp Ala Asp Arg Lys Ser Leu Glu Thr Lys
                   165                 170                 175
   Ser Leu Glu Val Thr Phe Thr Pro Val Ile Glu Asp Ile Gly Lys Val
   180 185 190
   Leu Val Cys Arg Ala Lys Leu His Ile Asp Glu Met Asp Ser Val Pro
   195 200 205
   Thr Val Arg Gln Ala Val Lys Glu Leu Gln Val Tyr Ile Ser Pro Lys
   210 215 220
   Asn Thr Val Ile Ser Val Asn Pro Ser Thr Lys Leu Gln Glu Gly Gly
   225 230 235 240
   Ser Val Thr Met Thr Cys Ser Ser Glu Gly Leu Pro Ala Pro Glu Ile
   245 250 255
   Phe Trp Ser Lys Lys Leu Asp Asn Gly Asn Leu Gln His Leu Ser Gly
   260 265 270
   Asn Ala Thr Leu Thr Leu Ile Ala Met Arg Met Glu Asp Ser Gly Ile
   275 280 285
   Tyr Val Cys Glu Gly Val Asn Leu Ile Gly Lys Asn Arg Lys Glu Val
   290 295 300
   Glu Leu Ile Val Gln Glu Lys Pro Phe Thr Val Glu Ile Ser Pro Gly
   305 310 315 320
   Pro Arg Ile Ala Ala Gln Ile Gly Asp Ser Val Met Leu Thr Cys Ser
   325 330 335
   Val Met Gly Cys Glu Ser Pro Ser Phe Ser Trp Arg Thr Gln Ile Asp
   340 345 350
   Ser Pro Leu Ser Gly Lys Val Arg Ser Glu Gly Thr Asn Ser Thr Leu
   355 360 365
   Thr Leu Ser Pro Val Ser Phe Glu Asn Glu His Ser Tyr Leu Cys Thr
   370 375 380
   Val Thr Cys Gly His Lys Lys Leu Glu Lys Gly Ile Gln Val Glu Leu
   385 390 395 400
   Tyr Ser Phe Pro Arg Asp Pro Glu Ile Glu Met Ser Gly Gly Leu Val
   405 410 415
   Asn Gly Ser Ser Val Thr Val Ser Cys Lys Val Pro Ser Val Tyr Pro
   420 425 430
   Leu Asp Arg Leu Glu Ile Glu Leu Leu Lys Gly Glu Thr Ile Leu Glu
   435 440 445
   Asn Ile Glu Phe Leu Glu Asp Thr Asp Met Lys Ser Leu Glu Asn Lys
   450 455 460
   Ser Leu Glu Met Thr Phe Ile Pro Thr Ile Glu Asp Thr Gly Lys Ala
   465 470 475 480
   Leu Val Cys Gln Ala Lys Leu His Ile Asp Asp Met Glu Phe Glu Pro
   485 490 495
   Lys Gln Arg Gln Ser Thr Gln Thr Leu Tyr Val Asn Val Ala Pro Arg
   500 505 510
   Asp Thr Thr Val Leu Val Ser Pro Ser Ser Ile Leu Glu Glu Gly Ser
   515 520 525
   Ser Val Asn Met Thr Cys Leu Ser Gln Gly Phe Pro Ala Pro Lys Ile
   530 535 540
   Leu Trp Ser Arg Gln Leu Pro Asn Gly Glu Leu Gln Pro Leu Ser Glu
   545 550 555 560
   Asn Ala Thr Leu Thr Leu Ile Ser Thr Lys Met Glu Asp Ser Gly Val
   565 570 575
   Tyr Leu Cys Glu Gly Ile Asn Gln Ala Gly Arg Ser Arg Lys Glu Val
   580 585 590
   Glu Leu Ile Ile Gln Val Thr Pro Lys Asp Ile Lys Leu Thr Ala Phe
   595 600 605
   Pro Ser Glu Ser Val Lys Glu Gly Asp Thr Val Ile Ile Ser Cys Thr
   610 615 620
   Cys Gly Asn Val Pro Glu Thr Trp Ile Ile Leu Lys Lys Lys Ala Glu
   625 630 635 640
   Thr Gly Asp Thr Val Leu Lys Ser Ile Asp Gly Ala Tyr Thr Ile Arg
   645 650 655
   Lys Ala Gln Leu Lys Asp Ala Gly Val Tyr Glu Cys Glu Ser Lys Asn
   660 665 670
   Lys Val Gly Ser Gln Leu Arg Ser Leu Thr Leu Asp Val Gln Gly Arg
   675 680 685
   Glu Asn Asn Lys Asp Tyr Phe Ser Pro Glu Leu Leu Val Leu Tyr Phe
   690 695 700
   Ala Ser Ser Leu Ile Ile Pro Ala Ile Gly Met Ile Ile Tyr Phe Ala
   705 710 715 720
   Arg Lys Ala Asn Met Lys Gly Ser Tyr Ser Leu Val Glu Ala Gln Lys
   725 730 735
   Ser Lys Val
<210> 2
   <211> 532
   <212> PRT
   <213> Homo sapiens
<400> 2
   Met Ala Pro Ser Ser Pro Arg Pro Ala Leu Pro Ala Leu Leu Val Leu
   1 5 10 15
   Leu Gly Ala Leu Phe Pro Gly Pro Gly Asn Ala Gln Thr Ser Val Ser
   20 25 30
   Pro Ser Lys Val Ile Leu Pro Arg Gly Gly Ser Val Leu Val Thr Cys
   35 40 45
   Ser Thr Ser Cys Asp Gln Pro Lys Leu Leu Gly Ile Glu Thr Pro Leu
   50 55 60
   Pro Lys Lys Glu Leu Leu Leu Pro Gly Asn Asn Arg Lys Val Tyr Glu
   65 70 75 80
   Leu Ser Asn Val Gln Glu Asp Ser Gln Pro Met Cys Tyr Ser Asn Cys
   85 90 95
   Pro Asp Gly Gln Ser Thr Ala Lys Thr Phe Leu Thr Val Tyr Trp Thr
   100 105 110
   Pro Glu Arg Val Glu Leu Ala Pro Leu Pro Ser Trp Gln Pro Val Gly
   115 120 125
   Lys Asn Leu Thr Leu Arg Cys Gln Val Glu Gly Gly Ala Pro Arg Ala
   130 135 140
   Asn Leu Thr Val Val Leu Leu Arg Gly Glu Lys Glu Leu Lys Arg Glu
   145                 150                 155                 160
   Pro Ala Val Gly Glu Pro Ala Glu Val Thr Thr Thr Val Leu Val Arg
                   165                 170                 175
   Arg Asp His His Gly Ala Asn Phe Ser Cys Arg Thr Glu Leu Asp Leu
               180                 185                 190
   Arg Pro Gln Gly Leu Glu Leu Phe Glu Asn Thr Ser Ala Pro Tyr Gln
           195                 200                 205
   Leu Gln Thr Phe Val Leu Pro Ala Thr Pro Pro Gln Leu Val Ser Pro
       210                 215                 220
   Arg Val Leu Glu Val Asp Thr Gln Gly Thr Val Val Cys Ser Leu Asp
   225                 230                 235                 240
   Gly Leu Phe Pro Val Ser Glu Ala Gln Val His Leu Ala Leu Gly Asp
                   245                 250                 255
   Gln Arg Leu Asn Pro Thr Val Thr Tyr Gly Asn Asp Ser Phe Ser Ala
               260                 265                 270
   Lys Ala Ser Val Ser Val Thr Ala Glu Asp Glu Gly Thr Gln Arg Leu
           275                 280                 285
   Thr Cys Ala Val Ile Leu Gly Asn Gln Ser Gln Glu Thr Leu Gln Thr
       290                 295                 300
   Val Thr Ile Tyr Ser Phe Pro Ala Pro Asn Val Ile Leu Thr Lys Pro
   305                 310                 315                 320
   Glu Val Ser Glu Gly Thr Glu Val Thr Val Lys Cys Glu Ala His Pro
                   325                 330                 335
   Arg Ala Lys Val Thr Leu Asn Gly Val Pro Ala Gln Pro Leu Gly Pro
               340                 345                 350
   Arg Ala Gln Leu Leu Leu Lys Ala Thr Pro Glu Asp Asn Gly Arg Ser
           355                 360                 365
   Phe Ser Cys Ser Ala Thr Leu Glu Val Ala Gly Gln Leu Ile His Lys
       370                 375                 380
   Asn Gln Thr Arg Glu Leu Arg Val Leu Tyr Gly Pro Arg Leu Asp Glu
   385                 390                 395                 400
   Arg Asp Cys Pro Gly Asn Trp Thr Trp Pro Glu Asn Ser Gln Gln Thr
                   405                 410                 415
   Pro Met Cys Gln Ala Trp Gly Asn Pro Leu Pro Glu Leu Lys Cys Leu
               420                 425                 430
   Lys Asp Gly Thr Phe Pro Leu Pro Ile Gly Glu Ser Val Thr Val Thr
           435                 440                 445
   Arg Asp Leu Glu Gly Thr Tyr Leu Cys Arg Ala Arg Ser Thr Gln Gly
       450                 455                 460
   Glu Val Thr Arg Lys Val Thr Val Asn Val Leu Ser Pro Arg Tyr Glu
   465                 470                 475                 480
   Ile Val Ile Ile Thr Val Val Ala Ala Ala Val Ile Met Gly Thr Ala
                   485                 490                 495
   Gly Leu Ser Thr Tyr Leu Tyr Asn Arg Gln Arg Lys Ile Lys Lys Tyr
               500                 505                 510
   Arg Leu Gln Gln Ala Gln Lys Gly Thr Pro Met Lys Pro Asn Thr Gln
           515                 520                 525
   Ala Thr Pro Pro
       530
<210> 3
   <211> 170
   <212> PRT
   <213> Homo sapiens
<400> 3
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Arg Arg Pro
       130                 135                 140
   Lys Gly Arg Gly Lys Arg Arg Arg Glu Lys Gln Arg Pro Thr Asp Cys
   145                 150                 155                 160
   His Leu Cys Gly Asp Ala Val Pro Arg Arg
                   165                 170
<210> 4
   <211> 149
   <212> PRT
   <213> Homo sapiens
<400> 4
   Met Pro Val Met Arg Leu Phe Pro Cys Phe Leu Gln Leu Leu Ala Gly
   1               5                   10                  15
   Leu Ala Leu Pro Ala Val Pro Pro Gln Gln Trp Ala Leu Ser Ala Gly
               20                  25                  30
   Asn Gly Ser Ser Glu Val Glu Val Val Pro Phe Gln Glu Val Trp Gly
           35                  40                  45
   Arg Ser Tyr Cys Arg Ala Leu Glu Arg Leu Val Asp Val Val Ser Glu
       50                  55                  60
   Tyr Pro Ser Glu Val Glu His Met Phe Ser Pro Ser Cys Val Ser Leu
   65                  70                  75                  80
   Leu Arg Cys Thr Gly Cys Cys Gly Asp Glu Asn Leu His Cys Val Pro
                   85                  90                  95
   Val Glu Thr Ala Asn Val Thr Met Gln Leu Leu Lys Ile Arg Ser Gly
               100                 105                 110
   Asp Arg Pro Ser Tyr Val Glu Leu Thr Phe Ser Gln His Val Arg Cys
           115                 120                 125
   Glu Cys Arg Pro Leu Arg Glu Lys Met Lys Pro Glu Arg Cys Gly Asp
       130                 135                 140
   Ala Val Pro Arg Arg
   145